# EUROPEAN PATENT APPLICATION

(11) **EP 4 726 029 A1**
(43) Date of publication of application: **15.04.2026**
(21) Application number: 24819310.4
(22) Date of filing: 04.06.2024
(51) Int. Cl.: C12N 5/077, A61K 31/085, A61P 3/04, A61P 3/06, C12N 1/00

(54) **ADIPOCYTE GROWTH INHIBITOR**

(30) Priority: 06.06.2023 JP 2023093018
(71) Applicant: Watanabe Oyster Laboratory Co., Ltd., Hachioji-Shi Tokyo 192-0154 (JP)
(72) Inventor: WATANABE, Mitsugu, Hachioji-shi, Tokyo 192-0154 (JP)
(74) Representative: Plougmann Vingtoft a/s
(86) International application number: PCT/JP2024/020345
(87) International publication number: WO 2024/253081

(57) **Abstract**

[Problem]

The present invention provides a useful treatment tool for obesity-related adipogenesis using DHMBA. DHMBA-containing culture suppresses the growth of 3T3-L1 preadipocytes in vitro, leading to a reduction in the number of cells. In an adipocyte differentiation process, culture in the presence of DHMBA suppresses adipogenesis in the process of differentiation of 3T3-L1 preadipocytes into adipocytes through the regulation of various signaling processes associated with insulin signaling.

[Solution]

The present invention includes 3,5-dihydroxy-4-methoxybenzyl alcohol (DHMBA) as an active ingredient and has an inhibitory action on 3T3-L1 preadipocyte growth.

## Description

### TECHNICAL FIELD

The present invention relates to an adipocyte growth inhibitor.

### BACKGROUND ART

An increase in adipocytes in adipose tissue leads to disordered pathophysiological conditions including type 2 diabetes, hyperlipidemia, hypertension, cardiovascular disease, and cancer. A phenolic antioxidant, 3,5-dihydroxy-4-methoxybenzyl alcohol (hereinafter also referred to as DHMBA), can control oxidative stress as intracellular radical scavenging. However, the role in the improvement and repair of the state of disease has not been fully understood.

The present invention has examined the regulatory effect of DHMBA on adipogenesis in mouse 3T3-L1 adipocytes in vitro. 3T3-L1 preadipocytes were cultured in a DMEM culture fluid containing 10% fetal bovine serum. Adding DHMBA to the culture fluid suppressed the proliferation of 3T3-L1 preadipocytes cultured in a culture fluid that did not contain cell differentiation factors into adipocytes.

Interestingly, when 3T3-L1 preadipocytes were cultured in a culture fluid containing insulin-containing differentiation-inducing factors, DHMBA did not affect the number of cells during the differentiation process of adipogenesis.

Culturing 3T3-L1 preadipocytes in a culture fluid containing DHMBA (1, 10, or 100 µM) suppressed lipid accumulation in adipocytes, and further, inhibited adipogenesis in 3T3-L1 adipocytes.

The strong inhibitory effect of DHMBA on adipogenesis was observed in the late stage of insulin-added culture. Adipogenesis was inhibited by the presence of Wortmannin, PD98059, or Bay 11-7082, which are inhibitors of pathways associated with insulin signaling pathways. In particular, the inhibitory effect of DHMBA on adipogenesis was also exerted in the presence of these inhibitors.

Furthermore, the exertion mechanism was associated with DHMBA reducing the levels of PPARγ and C/EBPα associated with the differentiation of preadipocytes and the levels of PI3 kinase 100α, Akt, MAPK, phosphorylated MAPK, and mTOR involved in the insulin signaling pathways.

### CITED DOCUMENTS

### PATENT LITERATURE

Patent Document 1: JP-A-2017-132753

### DISCLOSURE OF THE INVENTION

### PROBLEMS TO BE SOLVED BY THE INVENTION

The present invention provides a new strategy for obesity prevention and treatment, in which DHMBA suppresses adipogenesis through the regulation of various signaling pathways.

In general, obesity is caused by excessive energy resulting from the intake of high-calorie foods, and triglycerides are accumulated in adipocytes through fatty acids.

Increased adipocytes in adipose tissue lead to an abnormal increase in the level of hormones and cytokines secreted from that tissue and cause pathological conditions, such as type 2 diabetes, hyperlipidemia, hypertension, cardiovascular disease, and cancer. Obesity is now recognized as a risk factor for various diseases. Therefore, adipose tissue plays an important role in maintaining pathophysiological homeostasis.

Adipose tissue is composed of adipocytes formed by the differentiation of preadipocytes from bone marrow mesenchymal stem cells. Adipogenesis is an important process in the formation of adipocytes and the accumulation of lipids. As the main transcription factors that regulate this process, CCAAT/enhancer-binding protein alpha (C/EBPα), peroxisome proliferator-activated receptor gamma (PPARγ), sterol regulatory element-binding protein-1c (SREBP-1c), and fatty acid-binding protein (FABP4) are known. C/EBPα and PPARγ are indispensable for the differentiation of progenitor cells into mature adipocytes. PPARγ is involved in accelerating adipogenesis in cells lacking C/EBP expression. SREBP is an auxiliary regulatory factor of adipogenesis and plays an important role in regulating lipid metabolism. These transcription factors regulate the synthesis of fatty acids and triglycerides during adipogenesis. Furthermore, mitogen-activated protein kinase (MAPK) and protein kinase B (AKT), associated with insulin signaling pathways, are known to activate adipogenesis in adipocytes.

A new phenolic antioxidant, 3,5-dihydroxy-4-methoxybenzyl alcohol (DHMBA), was first found in Pacific oysters (Crassostrea Gigas). DHMBA has two properties to avoid oxidative stress as radical capture in some cells. DHMBA may play an important nutritional role in regulating the cell function as an antioxidant. Recently, DHMBA has been demonstrated to suppress the proliferation of metastatic prostate cancer cells by targeting a variety of signaling pathways, presenting a new strategy for prostate cancer treatment with DHMBA.

Furthermore, understanding the pharmacological effects of DHMBA is important for the prevention and treatment of various diseases.

The present invention was made to determine whether or not DHMBA regulates adipogenesis in 3T3-L1 preadipocytes in vitro. Here, it was found that DHMBA-containing culture suppresses the growth of 3T3-L1 preadipocytes in vitro, leading to a reduction in the number of cells. Such an effect was not induced in the differentiation process of adipocytes. It was found that in this cell differentiation process, culture in the presence of DHMBA suppresses adipogenesis in the process of differentiation of 3T3-L1 preadipocytes into adipocytes through the regulation of various signaling processes associated with insulin signaling. The present invention provides a useful treatment tool for obesity-related adipogenesis using DHMBA.

### SOLUTIONS TO THE PROBLEMS

The present invention is an adipocyte growth inhibitor including 3,5-dihydroxy-4-methoxybenzyl alcohol (DHMBA) as an active ingredient, the adipocyte growth inhibitor having an inhibitory action on 3T3-L1 preadipocyte growth,
alternatively, an inhibitor of lipid accumulation in an adipocyte, including 3,5-dihydroxy-4-methoxybenzyl alcohol (DHMBA) as an active ingredient, the inhibitor having an inhibitory action on lipid accumulation in an adipocyte,
alternatively, an adipocyte production inhibitor including 3,5-dihydroxy-4-methoxybenzyl alcohol (DHMBA) as an active ingredient, the adipocyte production inhibitor having an inhibitory action on adipocyte production,
alternatively, a regulator of a signaling pathway included in an adipocyte, including 3,5-dihydroxy-4-methoxybenzyl alcohol (DHMBA) as an active ingredient, the regulator having a regulatory action on a signaling pathway included in an adipocyte, or
alternatively, an agent for promoting a reduction of a protein level associated with a signaling process of adipogenesis in an adipocyte, the agent including 3,5-dihydroxy-4-methoxybenzyl alcohol (DHMBA) as an active ingredient and having a reduction action of reducing a protein level associated with a signaling process of adipogenesis in an adipocyte.

### EFFECTS OF THE INVENTION

The present invention provides an effect of being able to provide a new strategy for obesity prevention and treatment, in which DHMBA suppresses adipogenesis through the regulation of various signaling pathways.

That is, it was found that DHMBA-containing culture suppresses the growth of 3T3-L1 preadipocytes, leading to a reduction in the number of cells. In addition, it was found that in a cell differentiation process, culture in the presence of DHMBA suppresses adipogenesis in the process of differentiation of 3T3-L1 preadipocytes into adipocytes through the regulation of various signaling processes associated with insulin signaling.

Accordingly, the present invention provides an effect of being able to provide a useful treatment tool for obesity-related adipogenesis using DHMBA.

### BRIEF DESCRIPTION OF THE DRAWINGS

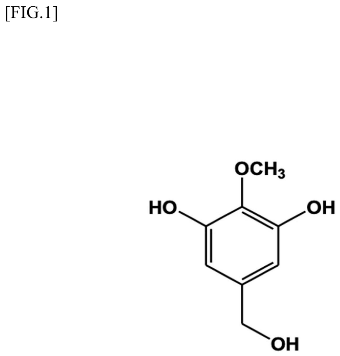

Fig. 1 illustrates the chemical structure of 3,5-dihydroxy-4-methoxybenzyl alcohol (DHMBA). The molecular formula for DHMBA is C₈H₁₀O₄, and its molecular weight is 170.164.

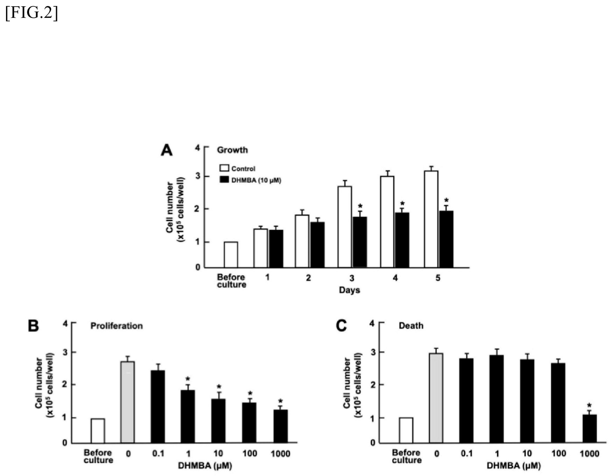

Fig. 2 is explanatory drawings for describing the actions of a marine factor 3,5-dihydroxy-4-methoxybenzyl alcohol (DHMBA) on cell proliferation or cell death of 3T3-L1 mouse embryo fibroblasts (preadipocytes). In the explanatory drawing of Fig. 2A, cells (1×10⁵ cells/ml per well of a 24-well plate) were cultured for one, two, three, four, or five days in a DMEM culture fluid containing 10% bovine calf serum (BCS) and 1% P/S in the presence of DHMBA (10 µM). In the explanatory drawing of Fig. 2B, in order to examine the effect of an increase in DHMBA concentration on cell proliferation, cells (1×10⁵ cells/ml per well of a 24-well plate) were cultured for three days in the presence of a vehicle (1% ethanol as the final concentration) or DHMBA (final concentration of 0.1, 1, 10, 100, or 1000 µM). In the explanatory drawing of Fig. 2C, in order to examine the effect of DHMBA on cell death, cells (1×10⁵ cells/ml per well of a 24-well plate) were cultured for three days. After the cells reached sub-confluence, DHMBA (final concentration of 0.1, 1, 10, 100, or 1000 µM) was added, and the cells were additionally cultured for 48 hours. After the culture, the number of cells adhered to a dish was counted. Data were shown as the mean ± SD (standard deviation) of values obtained from eight wells of the cell culture plate using different cell preparations. There was a significant difference, with p < 0.001, compared with the control group not containing DHMBA (gray bar). Significant difference tests were based on one-way analysis of variance with the Tukey-Kramer post-hoc test.

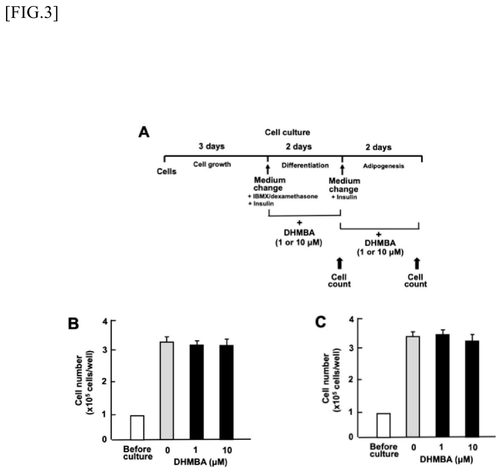

Fig. 3 is explanatory drawings for describing the actions of a marine factor 3,5-dihydroxy-4-methoxybenzyl alcohol (DHMBA) on the number of cells in a cell differentiation process of 3T3-L1 mouse preadipocytes. Fig. 3A illustrates a culture process of cells. In the explanatory drawing of Fig. 3B, cells (1×10⁵ cells/ml per well of a 24-well plate) were cultured for three days in DMEM containing 10% BCS and 1% P/S. Then, the culture fluid was replaced with a DMEM culture fluid containing cell differentiation-inducing factors of 0.5 mM IBMX, 1 µM dexamethasone, and insulin (10 µg/ml), a vehicle (1% ethanol as the final concentration) or DHMBA (1 or 10 µM) was added thereto, and the cells were cultured for two days. In the explanatory drawing of Fig. 3C, after another two days of culture (two days after the differentiation-inducing culture), the culture fluid was substituted with a culture fluid containing only insulin (100 µg/ml) without containing IBMX or dexamethasone, DHMBA (final concentration of 1 or 10 µM) was added thereto, and the cells were cultured for another two days.
After each culture, the number of cells adhered to a dish was counted. Data were shown as the mean ± SD of values obtained from eight wells of a total of two replication plates using different cell preparations. Significant difference tests were based on one-way analysis of variance with the Tukey-Kramer post-hoc test.

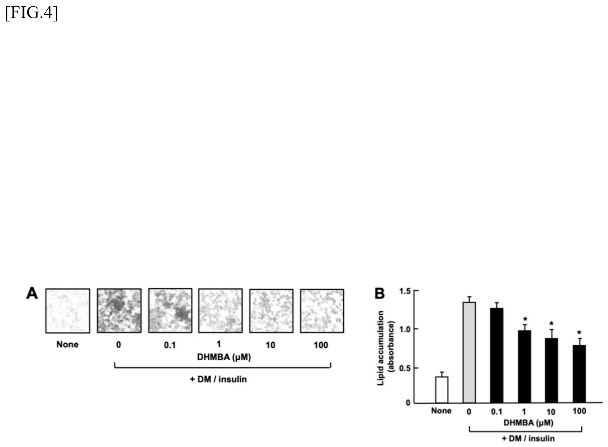

Fig. 4 is explanatory drawings for describing the actions of a marine factor 3,5-dihydroxy-4-methoxybenzyl alcohol (DHMBA) on fat accumulation in 3T3-L1 mouse preadipocytes.
   3T3-L1 preadipocytes (5×10⁴ cells/ml per well of a 24-well plate) were cultured for three days in a DMEM culture fluid containing 10% BCS and 1% P/S. On day 0 of the onset of cell differentiation, the cells that reached sub-confluence were used, the culture fluid was substituted with a differentiation factor-containing culture fluid containing 10% fetal bovine serum (FBS), 1% P/S, 0.5 mM IBMX, 1 µM dexamethasone, and insulin (10 µg/ml), and the cells were cultured for another two days. Two days after the cell differentiation-inducing culture, the culture fluid was replaced with a culture fluid (DMEM) containing fresh adipogenesis-promoting factors of 10% FBS and insulin (10 µg/ml) to further promote adipogenesis, and the cells were cultured for another two days. To quantify the effect of DHMBA on adipogenesis in 3T3-L1 cells, a vehicle (1% ethanol as the final concentration) or DHMBA (final concentration of 0.1, 1, 10, or 100 µM) was included in the differentiation culture fluid on day 0 of the differentiation culture and after two days. After the cell culture, the cells adhered to the 24-well plate were gently washed twice with PBS and fixed with 4% formaldehyde dissolved in PBS for 30 minutes. Afterward, the fixed cells were washed three times with PBS, and an Oil Red O solution (1.5% Oil Red O in isopropanol) was added to stain the cells for 60 minutes. In the explanatory drawing of Fig. 4A, after the staining, images of the stained fat accumulation cells were observed under a microscope and photographed. In the explanatory drawing of Fig. 4B, in order to quantify a lipid (triglyceride) content, the cells stained with Oil Red were extracted with isopropanol, and the absorbance of each well was measured at 510 nm using a spectrophotometer (µQuant, Bio-Tek Instruments). Data were shown as the mean ± SD of values obtained from eight wells of a total of two replication plates using different cell preparations. There was a significant difference, with p < 0.001, compared with the control group not containing DHMBA (gray bar). Significant difference tests were based on one-way analysis of variance with the Tukey-Kramer post-hoc test.

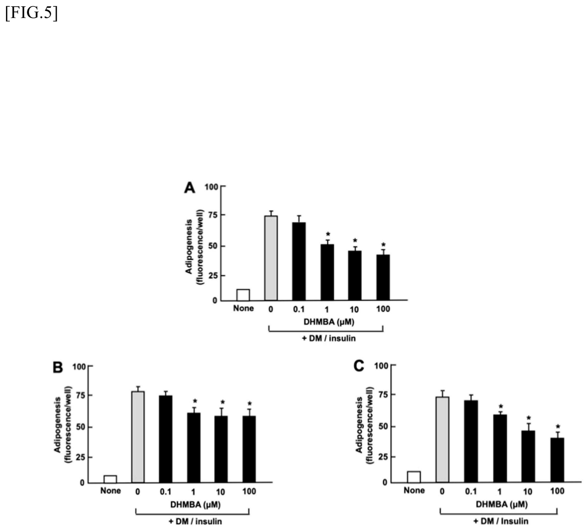

Fig. 5 is explanatory drawings for describing the actions of a marine factor 3,5-dihydroxy-4-methoxybenzyl alcohol (DHMBA) on adipogenesis in association with the differentiation of 3T3-L1 mouse preadipocytes. 3T3-L1 preadipocytes (1×10⁴ cells/ml per well of a 96-well plate) were cultured for three days, at which point they reached sub-confluence, in a DMEM culture fluid containing 10% BCS and 1% P/S. In the explanatory drawing of Fig. 5A, after the sub-confluence (on day 0 of differentiation culture), the culture fluid was substituted with a differentiation-inducing factor-containing DMEM culture fluid containing 10% fetal bovine serum (FBS), 1% P/S, 0.5 mM IBMX, 1 µM dexamethasone, and insulin, and a vehicle (1% ethanol as the final concentration) or DHMBA (final concentration of 0.1, 1, 10, or 100 µM) was added thereto. Two days later, the culture fluid was replaced with a culture fluid (DMEM) containing fresh 10% FBS and insulin (10 µg/ml), a vehicle (1% ethanol as the final concentration) or DHMBA (final concentration of 0.1, 1, 10, or 100 µM) was added thereto, and the cells were cultured for another two days. In the explanatory drawing of Fig. 5B, after the sub-confluence (on day 0 of differentiation culture), the culture fluid was substituted with a culture fluid containing 10% fetal bovine serum (FBS), 1% P/S, 0.5 mM IBMX, 1 µM dexamethasone, insulin (10 µg/ml), and DHMBA (0.1, 1, 10, or 100 µM), and the cells were cultured for another two days. Two days after the differentiation-inducing culture, the culture fluid was substituted with a culture fluid (not containing DHMBA) containing fresh 10% FBS and insulin (10 µg/ml), and the cells were cultured for another two days. In the explanatory drawing of Fig. 5C, after the sub-confluence (on day 0 of differentiation culture), the cells were cultured for two days in a differentiation-inducing factor-containing culture fluid containing 10% fetal bovine serum (FBS), 1% P/S, 0.5 mM IBMX, 1 µM dexamethasone, and insulin (10 µg/ml). Furthermore, two days after the differentiation-inducing culture, the culture fluid was substituted with a culture fluid containing fresh 10% FBS, insulin (10 µg/ml), and DHMBA (0.1, 1, 10, or 100 µM), and the cells were cultured for another two days. After the cell culture, the amount of intracellular lipid formation was measured using commercially available AdipoRed Assay Reagent (Lonza, Walkersville, MD, USA). Fluorescence was measured at an excitation of 485 nm and an emission of 570 nm with a 96-well plate reader. Data were shown as the mean ± SD of values obtained from eight wells of a total of two replication plates using different cell preparations. There was a significant difference, with p < 0.001, compared with the control group not containing DHMBA (gray bar). Significant difference tests were based on one-way analysis of variance with the Tukey-Kramer post-hoc test.

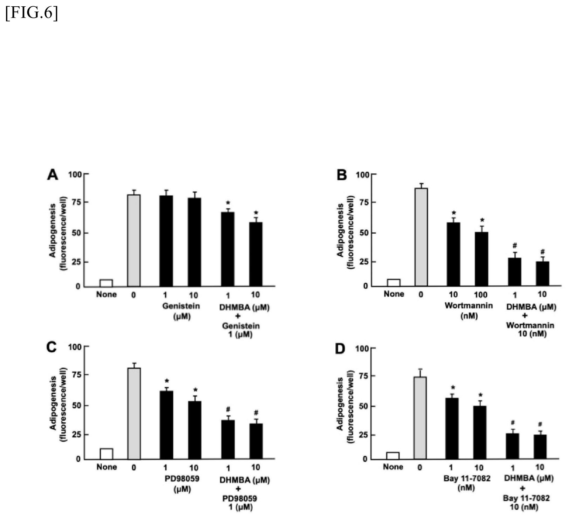

Fig. 6 is explanatory drawings for describing the actions of a marine factor 3,5-dihydroxy-4-methoxybenzyl alcohol (DHMBA) in the presence of various inhibitors of signaling pathways on adipogenesis in 3T3-L1 mouse preadipocytes. 3T3-L1 preadipocytes (1×10⁴ cells/ml per well of a 96-well plate) were cultured for three days, at which point they reached sub-confluence, in a DMEM culture fluid containing 10% BCS and 1% P/S. At the onset of cell differentiation culture after the sub-confluence (on day 0 of differentiation-inducing culture), the culture fluid was changed to a DMEM culture fluid containing 10% FBS, 1% P/S, 0.5 mM IBMX, 1 µM dexamethasone, and insulin (10 µg/ml), and the cells were additionally cultured for two days. Two days after the differentiation-inducing culture, the culture fluid was replaced with a fresh culture fluid containing genistein (1 or 10 µM) (Fig. 6A), Wortmannin (10 or 100 nM) (Fig. 6B), PD98059 (1 or 10 µM) (Fig. 6C), or Bay 11-7082 (1 or 10 nM) (Fig. 6D), a vehicle (1% ethanol as the final concentration) or DHMBA (final concentration of 1 or 10 µM) was added thereto, and the cells were cultured for another two days. After the cell culture, the amount of intracellular lipid triglyceride formation was measured using AdipoRed Assay Reagent (Lonza, Walkersville, MD, USA). Fluorescence was measured at an excitation of 485 nm and an emission of 570 nm with a 96-well plate reader. Data were shown as the mean ± SD of values obtained from eight wells of a total of two replication plates using different cell preparations. There was a significant difference, with p < 0.001, compared with the control group not containing DHMBA (gray bar). Significant difference tests were based on one-way analysis of variance with the Tukey-Kramer post-hoc test.

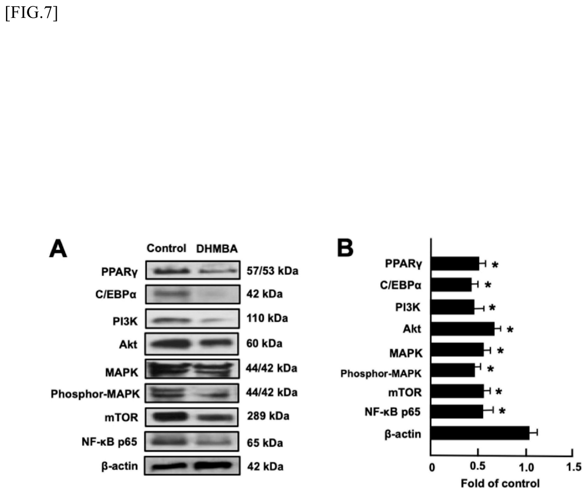

Fig. 7 is explanatory drawings for describing the actions of a marine factor 3,5-dihydroxy-4-methoxybenzyl alcohol (DHMBA) on the levels of various proteins associated with signaling of adipogenesis in adipocytes.
   3T3-L1 preadipocytes (1×10⁶ cells/100 mm dish, 10 ml culture fluid) were cultured for three days, at which point they reached sub-confluence, in a DMEM culture fluid containing 10% BCS and 1% P/S. At the onset of cell differentiation induction (on day 0 of differentiation induction), the culture fluid was replaced with a DMEM culture fluid containing 10% FBS, 1% P/S, 0.5 mM IBMX, and 1 µM dexamethasone, and the cells were additionally cultured for two days. Two days later, the culture fluid was replaced with a DMEM culture fluid containing 10% FBS, insulin (10 µg/ml), and DHMBA (final concentration of 10 µM), and the cells were cultured for another two days. After the culture, cell lysates were prepared. A protein sample of its centrifuged supernatant was used for western blot analysis, with 40 micrograms of the protein sample per lane. Representative data were shown in Fig. 7A. In Fig. 7B, bands were shown as multiples of controls (without DHMBA). Data were shown as the mean values ± SD obtained from a total of four films using different cell preparations. There was a significant difference, with p < 0.01, compared with the control group. Significant difference tests were based on one-way analysis of variance with the Tukey-Kramer post-hoc test.

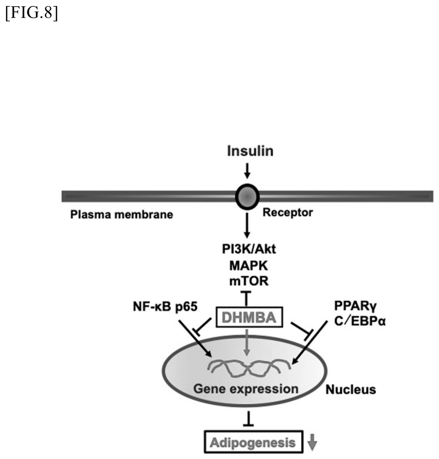

Fig. 8 is an explanatory drawing for describing the mechanism by which 3,5-dihydroxy-4-methoxybenzyl alcohol (DHMBA) inhibits adipogenesis in 3T3-L1 adipocytes. DHMBA reduced the levels of PPARγ and C/EBPα, which are transcription factors associated with the differentiation process of preadipocytes. In addition, DHMBA reduced the levels of PI3K, Akt, MAPK, phosphorylated MAPK, and mTOR, which are involved in the process of accelerating adipogenesis acceleration associated with insulin signaling. Furthermore, it was suggested that DHMBA also suppresses adipogenesis regulated by an NF-κB signaling pathway. Thus, DHMBA exerts an anti-adipogenesis effect by regulating various signaling pathways in adipocytes.

### DESCRIPTION OF PREFERRED EMBODIMENTS

### (Experimental Materials and Experimental Methods of the Present Invention)

### Reagents

A Dulbecco's Modified Eagle Medium (DMEM) containing 4.5 g/L glucose, L-glutamine, and sodium pyruvate, and an antibiotic drug (100 µg/mL penicillin and 100 µg/mL streptomycin; P/S) were obtained from Corning (Mediatech, Inc., Manassas, VA, USA). The antibiotic drug was contained at 1% in the DMEM. Fetal bovine serum (FBS) was obtained from Thermo Scientific HyClone (Logan, Utah). Bovine calf serum (BCS), dexamethasone, 3-isobutyl-1-methylxanthine (IBMX), Wortmannin, Bay 11-7082, and all other reagents were purchased from Sigma-Aldrich (St. Louis, Missouri).

### 3,5-Dihydroxy-4-Methoxybenzyl Alcohol (DHMBA)

A novel amphiphilic phenolic compound showing antioxidant properties, 3,5-dihydroxy-4-methoxybenzyl alcohol (DHMBA), was separated from Pacific oysters (Crassostrea Gigas). In the present invention, synthesized DHMBA was used. The structure of DHMBA was shown in Fig. 1. The purity of the synthesized DHMBA was 100%. The DHMBA was dissolved in 100% ethanol and stored at -20°C until it was used.

### 3T3-L1 Preadipocytes

3T3-L1 mouse embryo fibroblasts (preadipocytes) were obtained from American Type Culture Collection (Rockville, MD, USA). The cells were maintained in DMEM containing 10% BCS and 1% P/S under 5% CO₂ and 95% air at 37°C. The cells were cultured until they reached 70% to 80% confluence, and the culture fluid was replaced every other day. The cells were subcultured every three days.

### Assays for Cell Proliferation and Cell Death

In order to measure the progression of cell proliferation, 3T3-L1 preadipocytes (1×10⁵/ml per well) were cultured for one, two, three, or four days in a culture fluid containing a vehicle (1% ethanol as the final concentration) or DHMBA (10 µM) under 5% CO₂ and 95% air at 37°C. Furthermore, in an experiment to measure cell proliferation, 3T3-L1 preadipocytes (1 × 10⁵/ml per well of a 24-well plate) were cultured for three days in a culture fluid containing a vehicle (1% ethanol as the final concentration) or DHMBA (0.1, 1, 10, 100, or 1000 µM).

In an experiment to measure cell death, 3T3-L1 preadipocytes (1×10⁵/ml per well of a 24-well plate) were cultured for three days in a DMEM culture fluid containing 10% BCS and 1% P/S. The cells that reached sub-confluence were cultured for another 48 hours in the presence of a vehicle (1% ethanol as the final concentration) or DHMBA (0.1, 1, 10, 100, or 1000 µM) [15, 18] to examine the variation in the number of cells.

After the culture, adhering cells were separated from each well by adding an aseptic solution containing 0.05% trypsin and EDTA (0.1 ml per well) in PBS (Thermo Fisher Scientific, Waltham, MA, USA) not containing Ca²⁺/Mg²⁺ and incubating the cells for two minutes. Afterward, 0.9 ml of DMEM containing 10% FBS was added to each well to prepare cell suspensions [15, 17, 18]. In order to measure the number of living cells, a medium (0.1 ml) containing suspension cells was mixed with 0.1 ml of 0.5% trypan blue staining solution. The living cells were counted under a microscope (Olympus MTV-3) using a cell counter (Line Seiki H-102P, Tokyo, Japan) and a hemocytometer (Sigma-Aldrich, St. Louis, Missouri). Two counts were averaged in each measurement. The number of cells was shown as the number per well of a culture plate.

### Assay for Differentiation and Fat Accumulation of 3T3-L1 Preadipocytes

In order to understand the influence of DHMBA on the differentiation of 3T3-L1 preadipocytes, 3T3-L1 preadipocytes (5×10⁴ cells/ml per well of a 24-well plate) were cultured for three days, at which point they reached sub-confluence, in 10% bovine calf serum (BCS). After the cells reached sub-confluence (on day 0), the culture fluid was changed to a DMEM differentiation-inducing culture fluid containing 10% FBS, 1% P/S, 0.5 mM IBMX, 1 µM dexamethasone, and insulin (10 µg/ml), and the cells were cultured for another two days. Two days later, to promote adipogenesis, the culture fluid was replaced with a fresh differentiation culture fluid of DMEM containing 10% FBS and insulin (10 µg/ml), and the cells were cultured for another two days.

First, to quantify the effect of DHMBA on the number of cells, a vehicle (1% ethanol as the final concentration) or DHMBA (1 or 10 µM) was added to the differentiation-inducing culture fluid on day 0 of differentiation starting culture or after two days. After zero to two days of the differentiation culture or after two to four days of the culture, the number of cells adhered to a culture dish was measured as described in the previous section.

In an additional experiment to understand the effect of DHMBA on the content of lipids in 3T3-L1 adipocytes, a vehicle (1% ethanol as the final concentration) or DHMBA (0.1, 1, 10, or 100 µM) was included in the differentiation-inducing culture fluid to culture the cells from day 0 of the differentiation-inducing culture to four days later. In order to quantify the lipid content (triglycerides) after the cell culture, the cells in the 24-well plate were gently washed twice with PBS and fixed with 4% formaldehyde dissolved in PBS for 30 minutes. Subsequently, the fixed cells were washed three times with PBS and stained with an Oil Red O solution (1.5% Oil Red O dissolved in 100% isopropanol) for 60 minutes at room temperature [16]. After the staining, the stain solution was removed, and the cells were washed with PBS. Images of the stained lipid (triglyceride) drops were observed and photographed with a microscope (40 times) (Olympus IX71; Olympus Corporation, Tokyo, Japan). Furthermore, in order to quantify the lipid content, the Oil Red stain was extracted with isopropanol. 100% isopropanol (300 µl) was added to each well and shaken for 30 minutes, thereby eluting the stain. The eluate was used to measure the absorbance at 510 nm with a spectrophotometer (µQuant, Bio-Tek Instruments).

### Assay for Adipogenesis in 3T3-L1 Adipocytes

3T3-L1 preadipocytes (10⁴ cells/0.2 ml per well of a 96-well plate) were cultured for three days, at which point they reached sub-confluence, in DMEM containing 10% BCS and 1% P/S. After the sub-confluence (on day 0), the cells were cultured in a differentiation-inducing culture fluid containing 10% FBS, 1% P/S, 0.5 mM IBMX, and 1 µM dexamethasone with the inclusion of a vehicle (1% ethanol as the final concentration) or DHMBA (0.1, 1, 10, or 100 µM). Two days later, the culture fluid was replaced with a DMEM culture fluid containing insulin (10 µg/ml) without containing 10% FBS or DHMBA, and then, the cells were cultured for another two days.

In another experiment, the cells were cultured in a differentiation culture fluid not containing DHMBA on day 0 of differentiation-inducing culture. Two days later, the culture fluid was replaced with a DMEM culture fluid containing 10% FBS and insulin (10 µg/ml) that contained any of a vehicle (1% ethanol as the final concentration) and DHMBA (0.1, 1, 10, or 100 µM), and then, the cells were cultured for another two days.

In yet another experiment, to search for the involvement of intracellular signaling factors, the culture fluid was substituted with a culture fluid containing DMEM, which contained 10% FBS and insulin (10 µg/ml), and a vehicle (1% ethanol) or DHMBA (1 or 10 µM) two days after the differentiation culture. The cells were cultured for another two days in a culture fluid containing signaling inhibitory factors, such as genistein (1 or 10 µM), Wortmannin (10 or 100 nM), PD98059 (1 or 10 µM), or Bay 11-7082 (1 or 10 nM). After the cell culture, the amount of intracellular lipid (triglyceride) formation was quantified using AdipoRed Assay Reagent (Lonza, Walkersville, MD, USA) [19 to 21]. Fluorescence was measured at an excitation of 485 nm and an emission of 570 nm using a 96-well plate reader (µQuant, Bio-Tek Instruments).

### Western Blotting

3T3-L1 preadipocytes (1×10⁶ cells/100 mm dish used, in 10 ml of culture fluid) were cultured for three days, at which point they reached sub-confluence, in DMEM containing 10% BCS and 1% P/S. Then, the cells were cultured for two days in a DMEM differentiation culture fluid containing 10% FBS, 1% P/S, 0.5 mM IBMX, and 1 µM dexamethasone. Afterward, the culture fluid was replaced with a DMEM culture fluid containing 10% FBS and insulin (10 µg/ml) that contained or did not contain DHMBA (10 µM), and then, the cells were cultured for another two days. After the culture, the culture dish was washed three times with cold PBS (10 ml) to eliminate floating cells and dead cells, and adhering cells were scraped using a cell lysis solution containing protease and protein phosphatase inhibitors (Roche Diagnostics, Indianapolis, IN, USA). Next, the cell lysates were centrifuged for 10 minutes at 17,000 × g at 4°C. The concentration of proteins in the supernatant was quantified using Bio-Rad Protein Assay Dye (Bio-Rad Laboratories, Inc., Hercules, CA, USA) with bovine serum albumin as a standard. The extracted protein samples were stored at -80°C until they were used.

As indicated in the previous invention, a sample of 40 micrograms of supernatant proteins per lane was separated by SDS polyacrylamide gel electrophoresis (SDS-PAGE, 12%) and transferred to a PVDF membrane. Specific antibodies for target proteins, such as PPARγ (C26H12) (catalog No. 2435, rabbit antibody), C/EBPα (catalog No. 2295, rabbit), phosphoinositide 3-kinase p110α (PI3K; catalog No. 4255), Akt (catalog No. 9272, rabbit antibody), mitogen-activated protein kinase (MAPK; catalog No. 4695, rabbit antibody), phosphorylated MAPK (catalog No. 4695) 4370, rabbit antibody), mechanistic target of rapamycin (mTOR, catalog No. 4517, mouse antibody), NF-κB p65 (catalog No. 3034, rabbit antibody), and β-actin (catalog No. 3700, rabbit antibody), were obtained from Cell Signaling Technology (Danvers, MA, USA). The PVDF membrane was incubated overnight at 4°C using the above protein antibodies for immunoblotting.

After the incubation, the PVDF membrane was incubated at room temperature for 60 minutes in a horseradish peroxidase-conjugated secondary antibody (Santa Cruz Biotechnology, Inc., mouse sc-2005 or rabbit sc-2305; 1:1,000 dilution). Protein bands transferred to the PVDF membrane were detected on an X-ray film using a chemiluminescent substrate (catalog No. 34577, Thermo Scientific, Rockford, IL, USA). Four independent experiments were conducted for data, their films were scanned with an Epson Perfection 1660 photo scanner, and the bands were quantified using ImageJ2 software (National Institutes of Health, Bethesda, MD, USA).

### Statistical Analysis

Statistical significance was measured using GraphPad InStat version 3 for Windows XP (GraphPad Software Inc., La Jolla, CA). The data were shown as the mean values ± standard deviations (SD). Multiple comparisons were performed using one-way analysis of variance (ANOVA) with the Tukey-Kramer post-hoc multiple comparisons on parametric data. A p-value less than 0.05 was considered to be statistically significant.

### (Experimental Results)

### (Effect of DHMBA on Proliferation of 3T3-L1 Preadipocytes)

First, it was examined whether or not DHMBA affects the growth of 3T3-L1 preadipocytes (Fig. 2).

Fig. 2 is explanatory drawings for describing the actions of a marine factor 3,5-dihydroxy-4-methoxybenzyl alcohol (DHMBA) on cell proliferation or cell death of 3T3-L1 mouse embryo fibroblasts (preadipocytes). In the explanatory drawing of Fig. 2A, cells (1×10⁵ cells/ml per well of a 24-well plate) were cultured for one, two, three, four, or five days in a DMEM culture fluid containing 10% bovine calf serum (BCS) and 1% P/S in the presence of DHMBA (10 µM). In the explanatory drawing of Fig. 2B, in order to examine the effect of an increase in DHMBA concentration on cell proliferation, cells (1×10⁵ cells/ml per well of a 24-well plate) were cultured for three days in the presence of a vehicle (1% ethanol as the final concentration) or DHMBA (final concentration of 0.1, 1, 10, 100, or 1000 µM). In the explanatory drawing of Fig. 2C, in order to examine the effect of DHMBA on cell death, cells (1×10⁵ cells/ml per well of a 24-well plate) were cultured for three days. After the cells reached sub-confluence, DHMBA (final concentration of 0.1, 1, 10, 100, or 1000 µM) was added, and the cells were additionally cultured for 48 hours. After the culture, the number of cells adhered to a dish was counted. Data were shown as the mean ± SD (standard deviation) of values obtained from eight wells of the cell culture plate using different cell preparations. There was a significant difference, with p < 0.001, compared with the control group not containing DHMBA (gray bar). Significant difference tests were based on one-way analysis of variance with the Tukey-Kramer post-hoc test.

Cells (10⁵ cells/ml per well of a 24-well plate) were cultured for one, two, three, four, and five days in the presence of a vehicle (1% ethanol as the final concentration) or DHMBA (10 µM). Cell proliferation was suppressed by culture with DHMBA for three to five days. Therefore, it was found that DHMBA suppresses the growth of 3T3-L1 preadipocytes in vitro (Fig. 2A).

Next, the cells were cultured for three days in the presence of a culture fluid to which a vehicle or DHMBA with increased concentration (0.1, 1, 10, 100, or 1000 µM) was added. Cell proliferation was suppressed at concentrations ranging from 1 µM to 1000 µM DHMBA (Fig. 2B).

Furthermore, in order to examine the effect of DHMBA on cell death of 3T3-L1 preadipocytes, the cells were cultured for three days in DMEM containing 10% FBS and 1% P/S. After reaching sub-confluence, the cells were cultured for another two days in a culture fluid containing 10% FBS and 1% P/S with the inclusion of DHMBA (0.1, 1, 10, 100, or 1000 µM) (Fig. 2C). The number of cells was reduced by the presence of DHMBA (1000 µM). This indicated that the higher DHMBA concentration, the more cell death is caused.

### (Effect of DHMBA on Cell Proliferation in Association with Differentiation Process of 3T3-L1 Preadipocytes)

Next, the effect of DHMBA on cell proliferation in a differentiation process of 3T3-L1 preadipocytes was examined (Fig. 3).

Fig. 3 is explanatory drawings for describing the actions of a marine factor 3,5-dihydroxy-4-methoxybenzyl alcohol (DHMBA) on the number of cells in a cell differentiation process of 3T3-L1 mouse preadipocytes. Fig. 3A illustrates a culture process of cells. In the explanatory drawing of Fig. 3B, cells (1×10⁵ cells/ml per well of a 24-well plate) were cultured for three days in DMEM containing 10% BCS and 1% P/S. Then, the culture fluid was replaced with a DMEM culture fluid containing cell differentiation-inducing factors of 0.5 mM IBMX, 1 µM dexamethasone, and insulin (10 µg/ml), a vehicle (1% ethanol as the final concentration) or DHMBA (1 or 10 µM) was added thereto, and the cells were cultured for two days. In the explanatory drawing of Fig. 3C, after another two days of culture (two days after the differentiation-inducing culture), the culture fluid was substituted with a culture fluid containing only insulin (100 µg/ml) without containing IBMX or dexamethasone, DHMBA (final concentration of 1 or 10 µM) was added thereto, and the cells were cultured for another two days.

After each culture, the number of cells adhered to a dish was counted. Data were shown as the mean ± SD of values obtained from eight wells of a total of two replication plates using different cell preparations. Significant difference tests were based on one-way analysis of variance with the Tukey-Kramer post-hoc test.

The cells that reached sub-confluence after three days of culture (day 0) was cultured for another two days in a DMEM culture fluid containing differentiation-inducing factors of 10% FBS, 1% P/S, 0.5 mM IBMX, 1 µM dexamethasone, and insulin (10 µg/ml) with the inclusion of DHMBA (1 or 10 µM) (Fig. 3A). The number of cells was not varied by the presence of DHMBA (Fig. 3B). Furthermore, two days later, at which point adipogenesis was promoted, the culture fluid was replaced with a DMEM culture fluid containing insulin (10 µg/ml) that contained or did not contain DHMBA (1 or 10 µM), and the cells were cultured for another two days. The number of cells was not varied by the presence of DHMBA (Fig. 3C). It was found from these results that even the addition of DHMBA in the differentiation process of adipogenesis does not affect the number of cells.

### (DHMBA Inhibits Lipid Accumulation in 3T3-L1 Adipocytes)

Fig. 4 is explanatory drawings for describing the actions of a marine factor 3,5-dihydroxy-4-methoxybenzyl alcohol (DHMBA) on fat accumulation in 3T3-L1 mouse preadipocytes.

3T3-L1 preadipocytes (5×10⁴ cells/ml per well of a 24-well plate) were cultured for three days in a DMEM culture fluid containing 10% BCS and 1% P/S. On day 0 of the onset of cell differentiation, the cells that reached sub-confluence were used, the culture fluid was substituted with a differentiation factor-containing culture fluid containing 10% fetal bovine serum (FBS), 1% P/S, 0.5 mM IBMX, 1 µM dexamethasone, and insulin (10 µg/ml), and the cells were cultured for another two days. Two days after the cell differentiation-inducing culture, the culture fluid was replaced with a culture fluid (DMEM) containing fresh adipogenesis-promoting factors of 10% FBS and insulin (10 µg/ml) to further promote adipogenesis, and the cells were cultured for another two days. To quantify the effect of DHMBA on adipogenesis in 3T3-L1 cells, a vehicle (1% ethanol as the final concentration) or DHMBA (final concentration of 0.1, 1, 10, or 100 µM) was included in the differentiation culture fluid on day 0 of the differentiation culture and after two days. After the cell culture, the cells adhered to the 24-well plate were gently washed twice with PBS and fixed with 4% formaldehyde dissolved in PBS for 30 minutes. Afterward, the fixed cells were washed three times with PBS, and an Oil Red O solution (1.5% Oil Red O in isopropanol) was added to stain the cells for 60 minutes. In the explanatory drawing of Fig. 4A, after the staining, images of the stained fat accumulation cells were observed under a microscope and photographed. In the explanatory drawing of Fig. 4B, in order to quantify a lipid (triglyceride) content, the cells stained with Oil Red were extracted with isopropanol, and the absorbance of each well was measured at 510 nm using a spectrophotometer (µQuant, Bio-Tek Instruments). Data were shown as the mean ± SD of values obtained from eight wells of a total of two replication plates using different cell preparations. There was a significant difference, with p < 0.001, compared with the control group not containing DHMBA (gray bar). Significant difference tests were based on one-way analysis of variance with the Tukey-Kramer post-hoc test.

To understand the effect of DHMBA on lipid accumulation in 3T3-L1 adipocytes, on day 0 of the cell culture with the differentiation-inducing factor-containing culture fluid and after two days, a medium culture fluid (1% ethanol as the final concentration) or DHMBA (0.1, 1, 10, or 100 µM) was included to culture the cells. After the culture, fat (triglycerides) accumulated in the cells was stained with Oil Red O. Then, it was found that culture with DHMBA reduced lipid accumulation in 3T3-L1 adipocytes (Fig. 4A). Furthermore, when the lipid content in the cells was measured, its decrease was confirmed (Fig. 4B). These results suggested that DHMBA suppresses the accumulation of lipids in adipocytes.

### (DHMBA Suppresses Adipogenesis in 3T3-L1 Adipocytes)

It was examined whether or not the adipogenesis in 3T3-L1 adipocytes was altered by culture with DHMBA (Fig. 5).

Fig. 5 is explanatory drawings for describing the actions of a marine factor 3,5-dihydroxy-4-methoxybenzyl alcohol (DHMBA) on adipogenesis in association with the differentiation of 3T3-L1 mouse preadipocytes. 3T3-L1 preadipocytes (1×10⁴ cells/ml per well of a 96-well plate) were cultured for three days, at which point they reached sub-confluence, in a DMEM culture fluid containing 10% BCS and 1% P/S. In the explanatory drawing of Fig. 5A, after the sub-confluence (on day 0 of differentiation culture), the culture fluid was substituted with a differentiation-inducing factor-containing DMEM culture fluid containing 10% fetal bovine serum (FBS), 1% P/S, 0.5 mM IBMX, 1 µM dexamethasone, and insulin, and a vehicle (1% ethanol as the final concentration) or DHMBA (final concentration of 0.1, 1, 10, or 100 µM) was added thereto. Two days later, the culture fluid was replaced with a culture fluid (DMEM) containing fresh 10% FBS and insulin (10 µg/ml), a vehicle (1% ethanol as the final concentration) or DHMBA (final concentration of 0.1, 1, 10, or 100 µM) was added thereto, and the cells were cultured for another two days. In the explanatory drawing of Fig. 5B, after the sub-confluence (on day 0 of differentiation culture), the culture fluid was substituted with a culture fluid containing 10% fetal bovine serum (FBS), 1% P/S, 0.5 mM IBMX, 1 µM dexamethasone, insulin (10 µg/ml), and DHMBA (0.1, 1, 10, or 100 µM), and the cells were cultured for another two days. Two days after the differentiation-inducing culture, the culture fluid was substituted with a culture fluid (not containing DHMBA) containing fresh 10% FBS and insulin (10 µg/ml), and the cells were cultured for another two days. In the explanatory drawing of Fig. 5C, after the sub-confluence (on day 0 of differentiation culture), the cells were cultured for two days in a differentiation-inducing factor-containing culture fluid containing 10% fetal bovine serum (FBS), 1% P/S, 0.5 mM IBMX, 1 µM dexamethasone, and insulin (10 µg/ml). Furthermore, two days after the differentiation-inducing culture, the culture fluid was substituted with a culture fluid containing fresh 10% FBS, insulin (10 µg/ml), and DHMBA (0.1, 1, 10, or 100 µM), and the cells were cultured for another two days. After the cell culture, the amount of intracellular lipid formation was measured using commercially available AdipoRed Assay Reagent (Lonza, Walkersville, MD, USA). Fluorescence was measured at an excitation of 485 nm and an emission of 570 nm with a 96-well plate reader. Data were shown as the mean ± SD of values obtained from eight wells of a total of two replication plates using different cell preparations. There was a significant difference, with p < 0.001, compared with the control group not containing DHMBA (gray bar). Significant difference tests were based on one-way analysis of variance with the Tukey-Kramer post-hoc test.

The cells were cultured for three days in DMEM containing 10% BCS and 1% P/S. At the point when reaching sub-confluence, the cells were cultured for four days (zero to four days after the differentiation) in the presence of DHMBA (0.1, 1, 10, or 100 µM) (Fig. 5A). Culture with DHMBA (1, 10, or 100 µM) suppressed adipogenesis in 3T3-L1 adipocytes (Fig. 5A). Such suppression was also observed in cultures zero to two days or two to four days after the differentiation in the presence of DHMBA (1, 10, or 100 µM) (Figs. 5B and 5C). In particular, the inhibition of adipogenesis in 3T3-L1 adipocytes cultured in a DHMBA-containing culture fluid was noticeable in the late stage of the differentiation (after two to four days) (Fig. 5C). These results indicated that DHMBA strongly controls the process of promoting adipogenesis associated with insulin signaling in 3T3-L1 adipocytes.

### (Actions of DHMBA on Adipogenesis in 3T3-L1 Adipocytes Cultured with Inhibitors of Intracellular Signaling Pathways)

To understand the mechanism by which DHMBA suppresses adipogenesis in 3T3-L1 adipocytes, the involvement of intracellular signaling factors was examined (Fig. 6).

Fig. 6 is explanatory drawings for describing the actions of a marine factor 3,5-dihydroxy-4-methoxybenzyl alcohol (DHMBA) in the presence of various inhibitors of signaling pathways on adipogenesis in 3T3-L1 mouse preadipocytes. 3T3-L1 preadipocytes (1×10⁴ cells/ml per well of a 96-well plate) were cultured for three days, at which point they reached sub-confluence, in a DMEM culture fluid containing 10% BCS and 1% P/S. At the onset of cell differentiation culture after the sub-confluence (on day 0 of differentiation-inducing culture), the culture fluid was changed to a DMEM culture fluid containing 10% FBS, 1% P/S, 0.5 mM IBMX, 1 µM dexamethasone, and insulin (10 µg/ml), and the cells were additionally cultured for two days. Two days after the differentiation-inducing culture, the culture fluid was replaced with a fresh culture fluid containing genistein (1 or 10 µM) (Fig. 6A), Wortmannin (10 or 100 nM) (Fig. 6B), PD98059 (1 or 10 µM) (Fig. 6C), or Bay 11-7082 (1 or 10 nM) (Fig. 6D), a vehicle (1% ethanol as the final concentration) or DHMBA (final concentration of 1 or 10 µM) was added thereto, and the cells were cultured for another two days. After the cell culture, the amount of intracellular lipid triglyceride formation was measured using AdipoRed Assay Reagent (Lonza, Walkersville, MD, USA). Fluorescence was measured at an excitation of 485 nm and an emission of 570 nm with a 96-well plate reader. Data were shown as the mean ± SD of values obtained from eight wells of a total of two replication plates using different cell preparations. There was a significant difference, with p < 0.001, compared with the control group not containing DHMBA (gray bar). Significant difference tests were based on one-way analysis of variance with the Tukey-Kramer post-hoc test.

Two days after the cell differentiation, genistein (1 or 10 µM), Wortmannin (10 or 100 nM), PD98059 (1 or 10 µM), or Bay 11-7082 (1 or 10 nM) were added, and the cells were cultured for two days. While lipogenesis was not altered by the presence of genistein (Fig. 6A), DHMBA (1 or 10 µM) inhibited adipogenesis in the presence of genistein (Fig. 6A). In particular, adipogenesis was inhibited by the cultures with Wortmannin (10 or 100 nM) (Fig. 6B), PD98059 (1 or 10 µM) (Fig. 6C), or an NF-κB signaling inhibitor, Bay 11-7082 (1 or 10 nM) (Fig. 6D), associated with insulin signaling. These inhibitions were further enhanced by the presence of DHMBA (1 or 10 µM), suggesting that DHMBA suppresses adipogenesis by inhibiting a variety of pathways including insulin and NF-κB signaling.

### (Action of DHMBA on Protein Levels Associated with Signaling Process of Adipogenesis)

Furthermore, to understand the mechanism of action of DHMBA's inhibitory effect on adipogenesis in adipocytes, it was examined whether or not the levels of important proteins involved in adipogenesis are regulated by DHMBA (Figs. 7A and 7B).

Fig. 7 is explanatory drawings for describing the actions of a marine factor 3,5-dihydroxy-4-methoxybenzyl alcohol (DHMBA) on the levels of various proteins associated with signaling of adipogenesis in adipocytes.

3T3-L1 preadipocytes (1×10⁶ cells/100 mm dish, 10 ml culture fluid) were cultured for three days, at which point they reached sub-confluence, in a DMEM culture fluid containing 10% BCS and 1% P/S. At the onset of cell differentiation induction (on day 0 of differentiation induction), the culture fluid was replaced with a DMEM culture fluid containing 10% FBS, 1% P/S, 0.5 mM IBMX, and 1 µM dexamethasone, and the cells were additionally cultured for two days. Two days later, the culture fluid was replaced with a DMEM culture fluid containing 10% FBS, insulin (10 µg/ml), and DHMBA (final concentration of 10 µM), and the cells were cultured for another two days. After the culture, cell lysates were prepared. A protein sample of its centrifuged supernatant was used for western blot analysis, with 40 micrograms of the protein sample per lane. Representative data were shown in Fig. 7A. In Fig. 7B, bands were shown as multiples of controls (without DHMBA). Data were shown as the mean values ± SD obtained from a total of four films using different cell preparations. There was a significant difference, with p < 0.01, compared with the control group. Significant difference tests were based on one-way analysis of variance with the Tukey-Kramer post-hoc test.

Cell culture with the addition of DHMBA (10 µM) reduced the levels of PPARγ and C/EBPα, which are transcription factors associated with the differentiation of preadipocytes. In addition, DHMBA caused a reduction in the levels of PI3 kinase 100α, Akt, MAPK, phosphorylated MAPK, and mTOR, which are involved in insulin signaling pathways in the acceleration of lipogenesis. Furthermore, DHMBA reduced the level of a transcription factor, NF-κB p65. Therefore, it was found that culture with DHMBA reduces the levels of a variety of protein molecules associated with adipogenesis.

### (Discussion)

Adipose tissue is composed of adipocytes formed by the differentiation of bone marrow mesenchymal stem cells into preadipocytes. Adipose tissue plays an important role in maintaining pathophysiological homeostasis of type 2 diabetes, hyperlipidemia, hypertension, cardiovascular disease, cancer, and the like.

DHMBA has been proven to have antioxidative stress as radical capture in cells. In addition, the effect of DHMBA on adipogenesis was ascertained in the present invention. In the present invention, it was found that culturing 3T3-L1 preadipocytes in a DHMBA-containing culture fluid suppresses their cell growth. Furthermore, it was found that DHMBA controls adipogenesis in the differentiation process of preadipocytes without relying on the exertion of inhibitory effects on cell growth in vitro. The present invention presented a new view that DHMBA has inhibitory effects on the growth of adipocytes and adipogenesis.

Culture of adipocytes in the presence of DHMBA inhibited the promotion of adipogenesis associated with insulin signaling in 3T3-L1 adipocytes. The adipogenesis inhibitory effect of DHMBA was strongly exerted in the late stage of differentiation compared to the early stage of the differentiation process of 3T3-L1 preadipocytes. Early-stage culture was performed in a culture fluid containing differentiation-inducing factors of IBMX, dexamethasone, and insulin, while late-stage culture contained only insulin. In particular, DHMBA strongly inhibited adipogenesis in the late stage. Insulin exerts a strong promoting effect on adipogenesis and lipid accumulation in mature adipocytes. Therefore, DHMBA was considered to strongly control the process in which adipogenesis is enhanced by insulin.

The mechanism of signaling pathways in obesity is complex. In order to elucidate the mechanism by which culture of adipocytes in the presence of DHMBA suppresses adipogenesis, adipocytes were cultured in the presence of genistein, Wortmannin, PD98059, and Bay 11-7089, which are associated with insulin signaling pathways. Genistein is an inhibitor of tyrosine kinase. Wortmannin is an inhibitor of the PI3K/Akt signaling pathway. PD98059 is an inhibitor of ERK/MAP kinase. Bay 11-7089 is an inhibitor of NF-κB. Even in the presence of these inhibitors, the adipogenesis inhibitory effect of DHMBA was exerted. These results suggested that DHMBA strongly inhibits adipogenesis in 3T3-L1 adipocytes by suppressing various signaling processes associated with insulin signaling.

Furthermore, an experiment was attempted to clarify the mechanism by which DHMBA suppresses adipogenesis in adipocytes. It was found that culture of adipocytes with the addition of DHMBA reduces the levels of PPARγ and C/EBPα, which are transcription factors associated with the differentiation process of preadipocytes. These results supported the view that DHMBA inhibits the differentiation process of preadipocytes into adipocytes. Moreover, culture of adipocytes with the addition of DHMBA reduced the levels of PI3K, Akt, MAPK, phosphor-MAPK, and mTOR, which are molecules involved in the process of promoting adipogenesis associated with insulin signaling. Activation of the mTOR pathway accelerates the differentiation of 3T3-L1 preadipocytes. These results further supported the notion that DHMBA regulates the process of adipogenesis enhanced by insulin. Furthermore, DHMBA reduced the level of NF-κB p65 in adipocytes. Lipogenesis was suppressed by the presence of Bay 11-7089, which is an inhibitor of NF-κB signaling. In addition, culture with DHMBA inhibited adipogenesis in the presence of Bay 11-7089. The knowledge suggested that culture of adipocytes with the addition of DHMBA also suppresses the adipogenesis process that regulates an NF-κB signaling pathway.

Fig. 8 is an explanatory drawing for describing the mechanism by which 3,5-dihydroxy-4-methoxybenzyl alcohol (DHMBA) inhibits adipogenesis in 3T3-L1 adipocytes.

DHMBA reduced the levels of PPARγ and C/EBPα, which are transcription factors associated with the differentiation process of preadipocytes. In addition, DHMBA reduced the levels of PI3K, Akt, MAPK, phosphorylated MAPK, and mTOR, which are involved in the process of accelerating adipogenesis acceleration associated with insulin signaling. Furthermore, it was suggested that DHMBA also suppresses adipogenesis regulated by the NF-κB signaling pathway. Thus, DHMBA exerts an anti-adipogenesis effect by regulating various signaling pathways in adipocytes.

As summarized in Fig. 8, DHMBA was considered to exert an anti-adipogenesis effect by regulating various signaling pathways in adipocytes.

In conclusion, while DHMBA is known to develop antioxidative stress as radical scavenging in cells, the present invention has revealed new knowledge that DHMBA exerts an inhibitory effect on adipogenesis promotion associated with insulin signaling in mouse 3T3-L1 adipocytes in vitro. The effect of DHMBA was considered to be exerted through various pathways involved in insulin signaling. Supplementation of DHMBA as a supplement may play a role in obesity prevention and treatment by inhibiting adipogenesis in adipocytes.

## Claims

1. An adipocyte growth inhibitor comprising 3,5-dihydroxy-4-methoxybenzyl alcohol (DHMBA) as an active ingredient, the adipocyte growth inhibitor having an inhibitory action on 3T3-L1 preadipocyte growth.

2. An inhibitor of lipid accumulation in an adipocyte, comprising 3,5-dihydroxy-4-methoxybenzyl alcohol (DHMBA) as an active ingredient, the inhibitor having an inhibitory action on lipid accumulation in an adipocyte.

3. An adipocyte production inhibitor comprising 3,5-dihydroxy-4-methoxybenzyl alcohol (DHMBA) as an active ingredient, the adipocyte production inhibitor having an inhibitory action on adipocyte production.

4. A regulator of a signaling pathway included in an adipocyte, comprising 3,5-dihydroxy-4-methoxybenzyl alcohol (DHMBA) as an active ingredient, the regulator having a regulatory action on a signaling pathway included in an adipocyte.

5. An agent for promoting a reduction of a protein level associated with a signaling process of adipogenesis in an adipocyte, the agent comprising 3,5-dihydroxy-4-methoxybenzyl alcohol (DHMBA) as an active ingredient and having a reduction action of reducing a protein level associated with a signaling process of adipogenesis in an adipocyte.
